(19) Europäisches Patentamt European Patent Office Office européen des brevets

(11) **EP 4 736 835 A1**

(12) **EUROPEAN PATENT APPLICATION**
published in accordance with Art. 153(4) EPC

(43) Date of publication:
**06.05.2026 Bulletin 2026/19**

(21) Application number: **24832131.7**

(22) Date of filing: **28.06.2024**

(51) International Patent Classification (IPC):
*A61K 6/60* (2020.01)    *A61K 6/62* (2020.01)
*A61K 6/887* (2020.01)    *C08F 2/44* (2006.01)
*C08F 290/06* (2006.01)

(52) Cooperative Patent Classification (CPC):
**A61K 6/60; A61K 6/62; A61K 6/887; C08F 2/44;
C08F 290/06**

(86) International application number:
**PCT/JP2024/023620**

(87) International publication number:
**WO 2025/005274 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.06.2023 JP 2023107524**

(71) Applicant: **Kuraray Noritake Dental Inc.
Kurashiki-shi, Okayama 710-0801 (JP)**

(72) Inventor: **SUZUKI, Kenji
Tainai-shi, Niigata 959-2653 (JP)**

(74) Representative: **D Young & Co LLP
3 Noble Street
London EC2V 7BQ (GB)**

(54) **DENTAL POLYMERIZABLE COMPOSITION**

(57)    The present invention provides a dental polymerizable composition that exhibits excellent toughness and water resistance in the cured product while reducing leachables from the cured product. The present invention relates to a dental polymerizable composition that comprises a polyfunctional (meth)acrylic polymerizable compound (A), and a photopolymerization initiator (B) containing no phosphine oxide framework. Preferably, the photopolymerization initiator (B) containing no phosphine oxide framework comprises a Norrish Type II photopolymerization initiator. Preferably, the polyfunctional (meth)acrylic polymerizable compound (A) comprises a polyfunctional (meth)acrylic polymerizable compound (A)-I having a molecular weight of less than 1,000, and/or a polyfunctional (meth)acrylic polymerizable compound (A)-II having a molecular weight of 1,000 or more.

EP 4 736 835 A1

**Description**

TECHNICAL FIELD

The present invention relates to dental polymerizable compositions, particularly stereolithographic dental polymerizable compositions formulated with a large amount of photopolymerization initiator. More specifically, the invention relates to a dental polymerizable composition that allows for the formation of a cured product with superior strength, toughness, water resistance, and color tone while achieving excellent biological safety with reduced amounts of leachables from the cured product. The invention is suited for stereolithographic materials used in dentistry, with optimum suitability for dental occlusal splints, denture base materials, and appliances for the treatment of sleep apnea.

BACKGROUND ART

Dental polymerizable compositions are employed in a variety of applications, including restorative filling materials for tooth defects or carious cavities, adhesives for securing such restorative filling materials to teeth, metal or ceramic crown restoration materials called inlays or crowns, luting agents for securing such crown restoration materials to teeth, and dental materials such as those for dental occlusal splints or denture bases. Typically, dental polymerizable compositions contain (meth)acrylic polymerizable monomers, polymerization initiators, and, optionally, fillers such as inorganic compounds, in addition to other optional additives.

The polymerization initiators used in such applications include photopolymerization initiators and chemical polymerization initiators. Dental polymerizable compositions fall into the following broad categories: photopolymerizable compositions formulated with photopolymerization initiators, chemical polymerizable compositions formulated with chemical polymerization initiators, and compositions that exhibit both photopolymerizable and chemical polymerizable properties by incorporating both types of initiators. Photopolymerization initiators are indispensable in most dental materials.

Various proposals have been made concerning optical 3D fabrication (stereolithography), a method that produces three-dimensional shaped articles through a repeated procedure whereby liquid photocurable resin composition containing a photopolymerization initiator is cured into a thin layer under controlled application of necessary amounts of light energy, and another thin layer is cured on the cured layer under controlled application of light after supplying another portion of the liquid photocurable composition onto the previously formed cured layer.

Vat stereolithography is a technique typically used for optical fabrication of three-dimensional shaped articles. In this technique, a liquid photocurable composition is placed in a vat, and a computer-controlled ultraviolet laser is selectively applied to the surface of the liquid photocurable composition to cure it to a predetermined thickness, forming a cured layer with the desired pattern. Continuously, another cured layer is formed on the cured layer by applying an ultraviolet laser in the same manner to the liquid photocurable composition supplied onto the previously cured layer in an amount necessary to form a single layer. This layering process is repeated until it produces the final three-dimensional shaped article. In recent years, this technique has attracted great interest because it enables easy and precision production of the desired three-dimensional shaped article in a relatively short time period, even when the article has a very complex shape.

Three-dimensional shaped articles created through stereolithography are expanding their applications from mere concept models to test models, prototypes, and final products. The field of dental materials is thought to greatly benefit from stereolithography because dental occlusal splints, denture bases, and mouthpiece-like materials used for treating sleep disorders (for example, appliances for preventing bruxism or treating sleep apnea) require shapes that vary from patient to patient, aside from being complex in shape.

Some dental occlusal splints are fitted to adjust tooth alignment or jaw position as in so-called orthodontic mouthpieces or aligners while others are attached to teeth to reduce tooth wear due to clenching. Mouthguards are another type of splints that are worn in the mouth to protect the stomatognathic system and the brain by reducing injuries caused when large external forces are applied to teeth and jawbones during sports activities in contact sports. In orthodontics, the use of these devices has gained wide popularity over the last years due to their favorable aesthetics and convenient removability.

Denture base materials are materials used for the gum as a part of a denture attached to replace missing teeth. The demand for dentures has rapidly increased in recent years because of increasing ageing populations.

Appliances for the treatment of sleep apnea include appliances (oral appliances, or OA) attached to teeth during sleep for the treatment of obstructive sleep apnea syndrome (OSAS), and its use has been rapidly increasing.

Common requirements for dental materials, particularly, dental occlusal splints, denture base materials, and OA include strength, toughness, water resistance, and color tone. Low strength creates discomfort by increasing flexure or deformation during wear, whereas a loss of toughness necessitates frequent replacement as it increases susceptibility to breakage from biting forces or deformation during wear. A loss of water resistance causes a reduction of mechanical properties during use, making the appliance practically useless by making the appliance easily deformable or breakable while being worn.

Another consideration is that fabrication of dental occlusal splints, denture base materials, and appliances for the treatment of sleep apnea typically requires taking an impression of the oral cavity. This is seen as a problem because the procedure involves discomfort, and places a burden on patients, in addition to requiring high technical skills.

Recent advances in digital technology have led to approaches that make use of an intraoral optical scan for taking an oral impression, and there have been attempts to apply stereolithography techniques for shaping. For fabrication, a photocurable resin composition is used. As a rule, resin compositions that develop strength tend to be more brittle, and attempts to impart toughness (flexibility) to the resin composition while maintaining strength often result in a flexible molecular structure. This encourages moisture penetration, which can potentially lead to a serious problem involving not only reduced strength but also increased leaching of degradation products of the photopolymerization initiator. Particularly, dental materials employing stereolithographic materials require more photopolymerization initiator than non-stereolithographic dental materials because of the notably shorter exposure time to light. This higher photopolymerization initiator content can lead to yellowing. Problems related to leaching from the cured product, as well as color issues such as staining and yellowing are critical, and it has been challenging to adequately satisfy these properties along with other properties such as toughness and water resistance.

Against this backdrop, techniques are available that enable the production of a cured product that exhibits high safety with no bleed-out while achieving excellent mechanical characteristics and transparency. For example, Patent Literature 1 discloses a photopolymerizable resin composition that incorporates a hydrogen abstraction photopolymerization initiator having an ethylenically unsaturated group(s).

CITATION LIST

Patent Literature

Patent Literature 1: WO2023/074620

SUMMARY OF INVENTION

Technical Problem

However, the photopolymerization initiator described in Patent Literature 1 is not readily available, and also raises concerns about storage stability due to the presence of polymerizable groups. Furthermore, Patent Literature 1 makes no mention of the strength and toughness of the cured product of the photopolymerizable resin composition, and investigation by the present inventor revealed that there is room for improvement.

It is accordingly an object of the present invention to provide a dental polymerizable composition that exhibits excellent toughness and water resistance while reducing leachables, and a stereolithographic material comprising such a dental polymerizable composition.

Solution to Problem

The present invention includes the following.

[1] A dental polymerizable composition comprising a polyfunctional (meth)acrylic polymerizable compound (A), and a photopolymerization initiator (B) containing no phosphine oxide framework.

[2] The dental polymerizable composition according to [1], wherein the photopolymerization initiator (B) containing no phosphine oxide framework comprises a Norrish Type II photopolymerization initiator.

[3] The dental polymerizable composition according to [1] or [2], wherein the polyfunctional (meth)acrylic polymerizable compound (A) comprises a polyfunctional (meth)acrylic polymerizable compound (A)-I with a molecular weight of less than 1,000, and/or a polyfunctional (meth)acrylic polymerizable compound (A)-II with a molecular weight of 1,000 or more.

[4] The dental polymerizable composition according to [3], wherein the polyfunctional (meth)acrylic polymerizable compound (A) comprises a polyfunctional urethanized (meth)acrylic polymerizable compound (A)-I-1 that contains a urethane bond and has a molecular weight of less than 1,000, and/or a polyfunctional urethanized (meth)acrylic polymerizable compound (A)-II-1 that contains a urethane bond and has a molecular weight of 1,000 or more.

[5] The dental polymerizable composition according to [4], wherein the polyfunctional (meth)acrylic polymerizable compound (A)-I-1 comprises a (meth)acrylate that contains no polymeric structure within a single molecule.

[6] The dental polymerizable composition according to [4] or [5], wherein the polyfunctional urethanized (meth)acrylic polymerizable compound (A)-II-1 comprises a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

[7] The dental polymerizable composition according to any one of [4] to [6], wherein the polyfunctional urethanized (meth)acrylic polymerizable compound (A)-II-1 comprises a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

[8] The dental polymerizable composition according to any one of [1] to [7], wherein the photopolymerization initiator (B) containing no phosphine oxide framework comprises at least one selected from the group consisting of an α-aminoketone compound, an oxime ester compound, and a thioxanthone.

[9] The dental polymerizable composition according to any one of [1] to [8], which further comprises a monofunctional (meth)acrylic polymerizable compound (C).

[10] The dental polymerizable composition according to [9], wherein the monofunctional (meth)acrylic polymerizable compound (C) comprises a monofunctional (meth)acrylic polymerizable compound (C)-I containing a cyclic structure.

[11] The dental polymerizable composition according to [10], wherein the monofunctional (meth)acrylic polymerizable compound (C)-I comprises one or more aromatic rings.

[12] The dental polymerizable composition according to [10] or [11], wherein the monofunctional (meth)acrylic polymerizable compound (C)-I comprises a plurality of aromatic rings.

[13] A stereolithographic material comprising a dental polymerizable composition of any one of [1] to [12].

[14] A denture base material comprising a cured product of a stereolithographic material of [13].

[15] A dental occlusal splint comprising a cured product of a stereolithographic material of [13].

[16] A material for treating sleep disorder, comprising a cured product of a stereolithographic material of [13].

[17] A method for stereolithographically producing a three-dimensional shaped article using a dental polymerizable composition of any one of [1] to [12].

Advantageous Effects of Invention

A dental polymerizable composition of the present invention exhibits excellent toughness and water resistance in the cured product while reducing leachables from the cured product. This makes a dental polymerizable composition of the present invention suited for stereolithographic materials used in dentistry, particularly, dental materials (dental occlusal splints and denture base materials), as well as materials used for appliances for the treatment of sleep apnea.

A dental polymerizable composition of the present invention also exhibits excellent mechanical strength in the cured product.

DESCRIPTION OF EMBODIMENTS

The following describes the present invention in detail through embodiments.

In this specification, the upper limits and lower limits of numeric ranges (for example, ranges of contents of components, ranges of values calculated from components, and ranges of physical properties) can be appropriately combined. For example, in this specification, the lower and upper limits of numeric ranges presented in a stepwise fashion may be combined independently. As an example, in a statement such as "preferably 0.2 to 8.0 mass%, more preferably 0.5 to 5.0 mass%" for the same parameter, it is possible to combine the preferred lower limit (0.2 mass%) with the more preferred upper limit (5.0 mass%) to form a range of "0.2 to 5.0 mass%", or, alternatively, "0.5 to 8.0 mass%."

Concerning numeric ranges, it is also possible to define only the lower limit without specifying the upper limit, for example, such as "12 mass% or more" or "12.5 mass% or more", based on a statement "more preferably 12 to 28 mass%, even more preferably 12.5 to 25 mass%". Similarly, it is possible to define only the upper limit without specifying the lower limit, such as "28 mass% or less" or "25 mass% or less."

Unless otherwise specified, a numeric range stated simply as "30 to 70" represents the range of 30 or more and 70 or less. Furthermore, for example, when a numeric range is "25 mass% or more and 75 mass% or less", the boundary values of the numeric range may be chosen from any of "more than 25 mass%", "25 mass% or more", "75 mass% or less", and "less than 75 mass%."

Similarly, for example, from statements presented for the same parameter such as "more preferably 0.05 parts or more by mass, even more preferably 0.1 parts or more by mass" and "more preferably 15 parts or less by mass, even more preferably 10 parts or less by mass", it is possible to combine the more preferred lower limit (0.05 parts or more by mass) with the even more preferred upper limit (10 parts or less by mass) to form the range of "0.05 parts or more by mass and 10 parts or less by mass." Furthermore, as with the foregoing, it is also possible to specify only the lower limit as "0.05 parts or more by mass" or "0.1 parts or more by mass", and, similarly, only the upper limit as "15 parts or less by mass" or "10 parts or less by mass."

The numeric ranges described above are merely examples using mass percentages and parts by mass, and the same applies to molecular weight and the like.

As used herein, the term "polymerizable compound" is used to refer to a polymerizable compound polymerized by the photopolymerization initiator described below.

As used herein, the term "(meth)acryl" is intended to be inclusive of both methacryl and acryl, and the same applies to similar expressions such as "(meth)acrylate", "(meth)acrylic acid ester", "(meth)acrylamide", and "(meth) acryloyloxy."

As used herein, the term "(meth)acrylic polymerizable compound" is intended to be inclusive of both polymerizable compounds having a (meth)acryloxy group as a polymerizable group, and polymerizable compounds having a (meth) acrylamide group as a polymerizable group.

As used herein, "polyfunctional" means that the number of polymerizable groups exceeds one.

As used herein, "molecular weight" refers to a single value calculated from atomic weights when an oligomer or polymeric structure is absent. When an oligomer or polymeric structure is present, the term "molecular weight" refers to, unless otherwise specified, the weight-average molecular weight determined by gel permeation chromatography (GPC), expressed in terms of polystyrene equivalents.

A dental polymerizable composition of the present invention comprises a polyfunctional (meth)acrylic polymerizable compound (A), and a photopolymerization initiator (B) containing no phosphine oxide framework.

[Polyfunctional (Meth)Acrylic Polymerizable Compound (A)]

In a dental polymerizable composition of the present invention, the polyfunctional (meth)acrylic polymerizable compound (A) is used to impart curability in combination with the photopolymerization initiator (B) in the dental polymerizable composition, and to impart strength and toughness to the cured product.

Preferably, the polyfunctional (meth)acrylic polymerizable compound (A) comprises a polyfunctional (meth) acrylic polymerizable compound (A)-I with a molecular weight of less than 1,000 (hereinafter, also referred to simply as "polyfunctional (meth)acrylic polymerizable compound (A)-I"), and/or a polyfunctional (meth)acrylic polymerizable compound (A)-II with a molecular weight of 1,000 or more (hereinafter, also referred to simply as "polyfunctional (meth)acrylic polymerizable compound (A)-II"). The polyfunctional (meth)acrylic polymerizable compound (A)-I is used to impart especially strength to the cured product of the dental polymerizable composition, whereas the polyfunctional (meth)acrylic polymerizable compound (A)-II is used to impart especially toughness to the cured product of the dental polymerizable composition.

In view of superior water resistance, the polyfunctional (meth)acrylic polymerizable compound (A)-I with a molecular weight of less than 1,000 is preferably one that does not contain a polymeric structure within a single molecule, more preferably a (meth)acrylate that does not contain a polymeric structure within a single molecule. The absence of a polymeric structure within a single molecule leads to reduced content of repeating units of polar functional groups per molecule, which tends to improve water resistance.

Examples of the polymeric structure include polyester, polycarbonate, polyurethane, polyether, polyamide, polyimide, polyarylate, and polyacrylate.

Examples of polyfunctional (meth)acrylic polymerizable compound (A)-I include polyfunctional urethanized (meth)acrylic polymerizable compound (A)-I-1 containing a urethane bond (hereinafter, also referred to simply as "polyfunctional urethanized (meth)acrylic polymerizable compound (A)-I-1"), and polyfunctional (meth)acrylic polymerizable compound (A)-I-2 containing no urethane bond (hereinafter, also referred to simply as "polyfunctional (meth)acrylic polymerizable compound (A)-I-2"). In view of superior toughness of the cured product and allowing the urethane bond to also serve as a hydrogen donating structure and achieve even greater curability and a further reduction of leachables from the cured product, it is preferable to comprise a polyfunctional urethanized (meth)acrylic polymerizable compound (A)-I-1. More preferably, the polyfunctional urethanized (meth)acrylic polymerizable compound (A)-I-1 is a (meth)acrylate that does not contain a polymeric structure within a single molecule.

In view of superior toughness and water resistance of the cured product and allowing the urethane bond to also serve as a hydrogen donating structure and achieve even greater curability and a further reduction of leachables from the cured product, a certain preferred embodiment is, for example, a dental polymerizable composition in which the polyfunctional (meth)acrylic polymerizable compound (A)-I comprises a polyfunctional urethanized (meth)acrylic polymerizable compound (A)-I-1, and the polyfunctional urethanized (meth)acrylic polymerizable compound (A)-I-1 is a (meth) acrylate containing neither a polymeric structure nor a ring structure within a single molecule.

In this specification, examples of the ring structure include aromatic rings, alicyclic rings (carbon rings that are saturated or unsaturated but not aromatic), and heterocyclic rings.

The alicyclic rings and heterocyclic rings are not particularly limited, and may be five-membered rings or six-membered rings, for example.

As an example, the polyfunctional urethanized (meth)acrylic polymerizable compound (A)-I-1 can be synthesized with ease by an addition reaction of an isocyanate group-containing compound having an alkylene backbone or phenylene backbone with a (meth)acrylic compound having a hydroxyl group (-OH).

The compound having an isocyanate group, and the (meth)acrylic compound having a hydroxyl group (-OH) may be compounds identical or similar to those exemplified below in the production of polyfunctional urethanized (meth)acrylic polymerizable compound (A)-II-1.

The addition reaction may be performed using known methods and conditions, and is not particularly limited.

The polyfunctional (meth)acrylic polymerizable compound (A)-I-1 includes bifunctional (meth)acrylic polymerizable compounds, and tri- and higher-functional (meth)acrylic polymerizable compounds. However, in view of toughness of the cured product, preferred are bifunctional (meth)acrylic polymerizable compounds.

In view of superior toughness and water resistance of the cured product and allowing the urethane bond to also serve as a hydrogen donating structure and achieve even greater curability and a further reduction of leachables from the cured product, a certain preferred embodiment (X-1) is, for example, a dental polymerizable composition in which the polyfunctional (meth)acrylic polymerizable compound (A)-I comprises a polyfunctional urethanized (meth)acrylic polymerizable compound (A)-I-1, and the polyfunctional urethanized (meth)acrylic polymerizable compound (A)-I-1 is a di(meth)acrylate containing neither a polymeric structure nor a ring structure within a single molecule.

Another preferred embodiment (X-2) is, for example, a dental polymerizable composition in which the polyfunctional (meth)acrylic polymerizable compound (A) comprises a polyfunctional urethanized (meth)acrylic polymerizable compound (A)-I-1 that contains a urethane bond and has a molecular weight of less than 1,000, and a polyfunctional urethanized (meth)acrylic polymerizable compound (A)-II-1 that contains a urethane bond and has a molecular weight of 1,000 or more, and the polyfunctional urethanized (meth)acrylic polymerizable compound (A)-I-1 is a di(meth)acrylate that contains neither a polymeric structure nor a ring structure within a single molecule.

Yet another preferred embodiment (X-3) is, for example, a dental polymerizable composition of embodiment (X-2) in which the polyfunctional urethanized (meth)acrylic polymerizable compound (A)-II-1 containing a urethane bond and having a molecular weight of 1,000 or more is a (meth)acrylic polymerizable compound containing no ring structure.

In embodiments (X-2) and (X-3), the polyfunctional urethanized (meth)acrylic polymerizable compound (A)-I-1 containing a urethane bond and having a molecular weight of less than 1,000, and the polyfunctional urethanized (meth) acrylic polymerizable compound (A)-II-1 containing a urethane bond and having a molecular weight of 1,000 or more act with other constituents, and provide superior toughness and water resistance to the cured product. Additionally, the urethane bond can also serve as a hydrogen donating structure, achieving even greater curability and a further reduction of leachables from the cured product.

In any of the embodiments above, the ring structure may be any of the ring structures previously noted.

Examples of the polyfunctional urethanized (meth)acrylic polymerizable compound (A)-I-1 include polyfunctional urethanized (meth)acrylates such as 2,2,4-trimethylhexamethylenebis(2-carbamoyloxyethyl)dimethacrylate (commonly known as UDMA), 2,4-tolylenebis(2-carbamoyloxyethyl)di(meth)acrylate, N,N-(2,2,4-trimethylhexamethylene)bis [2-(aminocarboxy)propane-1,3-diol]tetra(meth)acrylate, bishydroxyethyl methacrylate-isophorone diurethane, and 2,4-tolylenebis(2-carbamoyloxyethyl)dimethacrylate. Other examples of the polyfunctional urethanized (meth)acrylic polymerizable compound (A)-I-1 include phenyl glycidyl ether (meth)acrylate hexamethylene diisocyanate urethane prepolymer, pentaerythritol tri(meth)acrylate hexamethylene diisocyanate urethane prepolymer, pentaerythritol tri(meth) acrylate toluene diisocyanate urethane prepolymer, and pentaerythritol tri(meth)acrylate isophorone diisocyanate urethane prepolymer. The polyfunctional (meth)acrylic polymerizable compound (A)-I may be used alone, or two or more thereof may be used in combination. In view of the strength and toughness of the shaped article, preferred among these are 2,2,4-trimethylhexamethylenebis(2-carbamoyloxyethyl)dimethacrylate, and phenyl glycidyl ether acrylate hexamethylene diisocyanate urethane prepolymer, more preferably 2,2,4-trimethylhexamethylenebis(2-carbamoyloxyethyl)dimethacrylate.

In view of providing even superior strength, toughness, and water resistance in the shaped article when combined with other components, the content of the polyfunctional (meth)acrylic polymerizable compound (A)-I is preferably 10 to 70 mass%, more preferably 20 to 65 mass%, even more preferably 25 to 60 mass%, particularly preferably 30 to 55 mass% in total 100 mass% of the polymerizable compounds.

In another certain preferred embodiment, in view of even superior strength of the shaped article, the content of the polyfunctional (meth)acrylic polymerizable compound (A)-I is preferably 20 to 80 mass%, more preferably 30 to 75 mass%, even more preferably 35 to 75 mass%, particularly preferably 40 to 70 mass% in total 100 mass% of the polyfunctional (meth)acrylic polymerizable compound (A).

In yet another certain preferred embodiment, in view of providing even superior strength, toughness, and water resistance in the shaped article when combined with other components, the content of the polyfunctional (meth)acrylic polymerizable compound (A)-I is preferably 10 to 70 mass%, more preferably 20 to 65 mass%, even more preferably 25 to 60 mass%, particularly preferably 30 to 55 mass% in total 100 mass% of the dental polymerizable composition.

In any of the embodiments above, in view of enabling a further reduction of leachables from the cured product, the content of polyfunctional (meth)acrylic polymerizable compound (A)-I can be read as referring to the content of polyfunctional urethanized (meth)acrylic polymerizable compound (A)-I-1.

In another certain preferred embodiment, in view of even superior strength of the shaped article and allowing the urethane bond to also serve as a hydrogen donating structure and achieve even greater curability and a further reduction of leachables from the cured product, the content of the polyfunctional urethanized (meth)acrylic polymerizable compound (A)-I-1 in polyfunctional (meth)acrylic polymerizable compound (A)-I is preferably 20 mass% or more, more preferably 40 mass% or more, even more preferably 60 mass% or more, particularly preferably 80 mass% or more in total 100 mass% of the polyfunctional (meth)acrylic polymerizable compound (A)-I.

In certain preferred embodiments, the content of the polyfunctional urethanized (meth)acrylic polymerizable compound (A)-I-1 in polyfunctional (meth)acrylic polymerizable compound (A)-I may be 100 mass%.

The polyfunctional (meth)acrylic polymerizable compound (A)-I-2 includes bifunctional (meth)acrylic polymerizable compounds, and tri- and higher-functional (meth)acrylic polymerizable compounds. However, in view of toughness of the cured product, preferred are bifunctional (meth)acrylic polymerizable compounds.

Examples of the polyfunctional (meth)acrylic polymerizable compound (A)-I-2 include 2,2-bis((meth)acryloyloxyphenyl)propane, 2,2-bis(4-(meth)acryloyloxyethoxyphenyl)propane, 2,2-bis(4-(meth)acryloyloxydiethoxyphenyl)propane, 2,2-bis(4-(meth)acryloyloxypropoxyphenyl)propane, 2,2-bis[4-(2-hydroxy-3-methacryloyloxypropoxy)phenyl]propane (commonly known as Bis-GMA), 2,2-bis[4-(2-hydroxy-3-acryloyloxypropoxy)phenyl]propane, 2,2-bis(4-(meth)acryloyloxytetraethoxyphenyl)propane, 2,2-bis(4-(meth)acryloyloxypentaethoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxyisopropoxyphenyl)propane, 2,2-bis(4-(meth)acryloyloxypolyethoxyphenyl)propane (for example, with an average of 2.6 moles of ethoxy group added), 1,4-bis(2-(meth)acryloyloxyethyl)pyromellitate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and tricyclodecane dimethanol di(meth)acrylate.

Examples of the tri- and higher-functional polymerizable compounds include trimethylolpropane tri(meth)acrylate, trimethylolethane tri(meth)acrylate, trimethylol methane tri(meth)acrylate, pentaerythritol tri(meth)acrylate, pentaerythritol tetra(meth)acrylate, dipentaerythritol penta(meth)acrylate, and 1,7-di(meth)acryloyloxy-2,2,6,6-tetra(meth)acryloyloxymethyl-4-oxaheptane.

The polyfunctional urethanized (meth)acrylic polymerizable compound (A)-I requires a molecular weight of less than 1,000. In view of strength and toughness, the preferred molecular weight is 250 or more and 900 or less, more preferably 300 or more and 800 or less.

In a dental polymerizable composition of the present invention, the polyfunctional (meth)acrylic polymerizable compound (A)-II with a molecular weight of 1,000 or more (hereinafter, also referred to simply as "polyfunctional (meth)acrylic polymerizable compound (A)-II") is used to impart toughness to the cured product.

The polyfunctional (meth)acrylic polymerizable compound (A)-II can be classified into a polyfunctional (meth)acrylic polymerizable compound that contains an oligomer or polymeric structure within a single molecule, and a polyfunctional (meth)acrylic polymerizable compound that does not contain an oligomer or polymeric structure within a single molecule. However, in view of providing superior strength and toughness to the shaped article when combined with other components, it is preferable to comprise a polyfunctional (meth)acrylic polymerizable compound that contains an oligomer or polymeric structure within a single molecule.

The polyfunctional (meth)acrylic polymerizable compound (A)-II includes a polyfunctional urethanized (meth)acrylic polymerizable compound (A)-II-1 containing a urethane bond (hereinafter, also referred to simply as "polyfunctional urethanized (meth)acrylic polymerizable compound (A)-II-1"), and a polyfunctional (meth)acrylic polymerizable compound (A)-II-2 containing no urethane bond. However, in view of superior toughness of the cured product and allowing the urethane bond to also serve as a hydrogen donating structure and achieve even greater curability and a further reduction of leachables from the cured product, it is preferable to comprise a polyfunctional urethanized (meth)acrylic polymerizable compound (A)-II-1, more preferably a polyfunctional urethanized (meth)acrylic polymerizable compound (A)-II-1 containing an oligomer or polymeric structure within a single molecule.

In view of superior toughness of the cured product and allowing the urethane bond to also serve as a hydrogen donating structure and achieve even greater curability and a further reduction of leachables from the cured product, a certain preferred embodiment is, for example, a dental polymerizable composition that comprises a polyfunctional urethanized (meth)acrylic polymerizable compound (A)-I-1, and/or a polyfunctional urethanized (meth)acrylic polymerizable compound (A)-II-1.

The polyfunctional urethanized (meth)acrylic polymerizable compound (A)-II-1 containing an oligomer or polymeric structure within a single molecule can be easily synthesized through an addition reaction between a polyol having a polymer backbone (e.g., a polymeric structure such as a polyester, a polycarbonate, a polyurethane, a polyether, a polydiene, and a hydrogenated polydiene), a compound having an isocyanate group (-NCO), and a (meth)acrylic compound having a hydroxyl group (-OH). The polyfunctional urethanized (meth)acrylic polymerizable compound (A)-II-1 can also be easily synthesized by allowing lactone or alkylene oxide to undergo a ring-opening addition reaction with a (meth)acrylic compound having a hydroxyl group, and causing the resulting compound with a terminal hydroxyl group to undergo an addition reaction with a compound having an isocyanate group.

Preferably, the polyfunctional urethanized (meth)acrylic polymerizable compound (A)-II-1 is a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

In these structures, examples of the polyester include copolymers of dicarboxylic acids (for example, aromatic dicarboxylic acids such as phthalic acid and isophthalic acid, and unsaturated aliphatic dicarboxylic acids such as maleic acid) and aliphatic diols having 2 to 18 carbon atoms, copolymers of dicarboxylic acids (for example, saturated aliphatic dicarboxylic acids such as adipic acid and sebacic acid) and aliphatic diols having 2 to 18 carbon atoms, β-propiolactone polymers, γ-butyrolactone polymers, δ-valerolactone polymers, ε-caprolactone polymers, and copolymers of these. Preferred are copolymers of dicarboxylic acids (preferably, aromatic dicarboxylic acids, and unsaturated aliphatic dicarboxylic acids) and aliphatic diols having 2 to 12 carbon atoms, and copolymers of dicarboxylic acids (preferably, saturated aliphatic dicarboxylic acids) and aliphatic glycols having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior toughness and water resistance, preferred among these are the polyester structures.

In view of even superior toughness and water resistance, the polyfunctional urethanized (meth)acrylic polymerizable compound (A)-II-1 is preferably a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

Examples of the polyester include copolymers with a structure derived from a C4 to C18 aliphatic chain diol unit having a branched structure, and with a structure derived from a C4 to C18 aliphatic chain dicarboxylic acid and/or aromatic dicarboxylic acid unit having no branched structure.

Examples of the polycarbonate include copolymers with a structure derived from a C4 to C18 aliphatic chain diol unit having a branched structure, and with a structure derived from a C4 to C18 aliphatic chain diol unit having no branched structure.

Examples of the polyurethane include structures derived from a C4 to C18 aliphatic chain diol unit having a branched structure, and polycondensates of diisocyanate compounds.

Examples of the polyether include polyethers with a structure derived from a C4 to C18 aliphatic chain diol unit having a branched structure, and polyethers with a structure derived from a C4 to C18 aliphatic chain diol unit having no branched structure.

Examples of the poly-conjugated diene include homopolymers or copolymers of conjugated diene monomers. Examples of the conjugated diene monomers include 1,3-butadiene, isoprene, 2,3-dimethyl-1,3-butadiene, 1,3-pentadiene, and 1,3-hexadiene.

Examples of the hydrogenated poly-conjugated diene include hydrogenated polybutadiene, hydrogenated polyisoprene, and hydrogenated polyisobutylene.

Among these, in view of superior toughness and water resistance, it is preferable to comprise, as a polymer backbone, at least one structure selected from the group consisting of a polyester, a polycarbonate, a polyether, and a hydrogenated poly-conjugated diene, more preferably at least one structure selected from the group consisting of a polyester and a polycarbonate, even more preferably at least one structure selected from the group consisting of polyesters.

Examples of the diols constituting the C4 to C18 aliphatic chain diol unit having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a dental polymerizable composition having superior curability and low viscosity, preferred for use as polyols are C5 to C12 aliphatic diols having a methyl-group side chain, for example, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol, more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-(2-hydroxy-3-(meth)acryloyloxypropoxy)phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and hydroxy(meth)acrylamide compounds such as N-hydroxyethyl(meth)acrylamide, and N,N-bis(2-hydroxyethyl)(meth)acrylamide.

When the desired polyfunctional urethanized (meth)acrylic polymerizable compound (A)-II-1 is a (meth)acrylate compound, it can be produced by selecting a hydroxy(meth)acrylate compound.

The addition reaction between a compound having an isocyanate group and a (meth)acrylic compound having a hydroxyl group may be performed using known methods and conditions, and is not particularly limited.

Examples of the polyfunctional urethanized (meth)acrylic polymerizable compound (A)-II-1 with no polymeric structure within a single molecule include dipentaerythritol penta(meth)acrylate hexamethylene diisocyanate urethane prepolymer.

The polyfunctional urethanized (meth)acrylic polymerizable compound (A)-II-1 requires a molecular weight of 1,000 or more. In view of strength and toughness, the preferred molecular weight is 1,500 to 5,000, more preferably 2,000 to 3,000.

Examples of the polyfunctional urethanized (meth)acrylic polymerizable compound (A)-II include bifunctional (meth)acrylic polymerizable compounds, and tri- and higher-functional (meth)acrylic polymerizable compounds.

In a certain preferred embodiment, in view of providing even superior strength, toughness, and water resistance to the shaped article when combined with other components and allowing the urethane bond to also serve as a hydrogen donating structure and achieve even greater curability and a further reduction of leachables from the cured product, the content of the polyfunctional urethanized (meth)acrylic polymerizable compound (A)-II-1 is preferably 1 to 70 mass%, more preferably 5 to 60 mass%, even more preferably 10 to 50 mass%, particularly preferably 20 to 40 mass% in total 100 mass% of the polymerizable compounds.

In another certain preferred embodiment, in view of providing even superior strength, toughness, and water resistance to the shaped article when combined with other components and allowing the urethane bond to also serve as a hydrogen donating structure and achieve even greater curability and a further reduction of leachables from the cured product, the content of the polyfunctional urethanized (meth)acrylic polymerizable compound (A)-II-1 is preferably 10 to 75 mass%, more preferably 15 to 70 mass%, even more preferably 20 to 65 mass%, particularly preferably 25 to 60 mass% in total 100 mass% of the polyfunctional (meth)acrylic polymerizable compound (A).

In yet another certain preferred embodiment, in view of providing even superior strength, toughness, and water resistance to the shaped article when combined with other components and allowing the urethane bond to also serve as a hydrogen donating structure and achieve even greater curability and a further reduction of leachables from the cured product, the content of the polyfunctional urethanized (meth)acrylic polymerizable compound (A)-II-1 is preferably 1 to 70 mass%, more preferably 5 to 60 mass%, even more preferably 10 to 50 mass%, particularly preferably 20 to 40 mass% in total 100 mass% of the dental polymerizable composition.

In view of providing even superior strength, toughness, and water resistance to the cured product when combined with other components and allowing the urethane bond to also serve as a hydrogen donating structure and achieve even greater curability and a further reduction of leachables from the cured product, the content of the polyfunctional urethanized (meth)acrylic polymerizable compound (A)-II-1 is preferably 20 mass% or more, more preferably 40 mass% or more, even more preferably 60 mass% or more, particularly preferably 80 mass% or more in total 100 mass% of polyfunctional (meth)acrylic polymerizable compound (A)-II.

The content of polyfunctional urethanized (meth)acrylic polymerizable compound (A)-II-1 in polyfunctional (meth)acrylic polymerizable compound (A)-II may be 100 mass%.

The polyfunctional (meth)acrylic polymerizable compound (A)-II-2 with no urethane bond includes bifunctional (meth)acrylic polymerizable compounds, and tri- and higher-functional (meth)acrylic polymerizable compounds.

Examples of the polyfunctional (meth)acrylic polymerizable compound (A)-II-2 with no urethane bond include 2,2-bis(4-(meth)acryloyloxypolyethoxyphenyl)propane (for example, with an average of 25 moles or more of ethoxy group added), and polyethylene glycol di(meth)acrylate.

In view of providing even superior strength, toughness, and water resistance to the cured product when combined with other components, the content of the polyfunctional (meth)acrylic polymerizable compound (A)-II in a dental polymerizable composition of the present invention is preferably 1 to 80 mass%, more preferably 5 to 70 mass%, even more preferably 10 to 60 mass%, particularly preferably 20 to 50 mass% in total 100 mass% of the polymerizable compounds. The polyfunctional (meth)acrylic polymerizable compound (A)-II may be used alone, or two or more thereof may be used in combination.

In view of providing even superior strength, toughness, and water resistance to the cured product when combined with other components, the content of the polyfunctional (meth)acrylic polymerizable compound (A)-II in a dental polymerizable composition of the present invention is preferably 10 to 60 mass%, more preferably 15 to 65 mass%, even more preferably 20 to 50 mass%, particularly preferably 25 to 45 mass% in total 100 mass% of the dental polymerizable composition.

In view of even superior strength, toughness, and water resistance of the cured product, the mass ratio of the polyfunctional (meth)acrylic polymerizable compound (A)-I and polyfunctional (meth)acrylic polymerizable compound (A)-II in a dental polymerizable composition of the present invention is preferably (A)-I:(A)-II = 5:95 to 95:5, more preferably 10:90 to 90:10, even more preferably 20:80 to 80:20.

The content of the polyfunctional (meth)acrylic polymerizable compound (A) in a dental polymerizable composition of the present invention is preferably 10 to 95 mass% in total 100 mass% of the polymerizable compounds. In view of providing even superior strength, toughness, water resistance, and impact resistance to the shaped article when combined with other components, the content is more preferably 20 to 92 mass%, even more preferably 30 to 90 mass%, particularly preferably 40 to 85 mass%. The polyfunctional (meth)acrylic polymerizable compound (A) may be used alone, or two or more thereof may be used in combination.

In view of providing even superior strength, toughness, and water resistance to the shaped article when combined with other components, the content of the polyfunctional (meth)acrylic polymerizable compound (A) in a dental polymerizable composition of the present invention is preferably 10 to 95 mass%, more preferably 20 to 92 mass%, even more preferably 30 to 90 mass%, particularly preferably 40 to 85 mass% in total 100 mass% of the dental polymerizable composition.

[Monofunctional (Meth)Acrylic Polymerizable Compound (C)]

A certain preferred embodiment is, for example, a dental polymerizable composition that further comprises a monofunctional (meth)acrylic polymerizable compound (C).

In a dental polymerizable composition of the present invention, the monofunctional (meth)acrylic polymerizable compound (C) is used to reduce the viscosity of the dental polymerizable composition, and to impart greater water resistance to the cured product.

The monofunctional (meth)acrylic polymerizable compound (C) includes a monofunctional (meth)acrylic polymerizable compound (C)-I containing a cyclic structure, and a monofunctional (meth)acrylic polymerizable compound (C)-II containing no cyclic structure. In view of greater curability of the dental polymerizable composition and even superior water resistance of the cured product, preferred is monofunctional (meth)acrylic polymerizable compound (C)-I containing a cyclic structure.

Examples of the rings constituting the cyclic structure include aromatic rings, alicyclic rings, and heterocyclic rings (for example, such as nitrogen-containing heterocyclic rings).

The monofunctional (meth)acrylic polymerizable compound (C)-I containing a cyclic structure contains one or more rings (for example, aromatic rings), preferably a plurality of rings (two or more).

Examples of the monofunctional (meth)acrylic polymerizable compound (C)-I include (meth)acrylic acid ester compounds containing an aromatic ring, alicyclic (meth)acrylic acid ester compounds, cyclic (meth)acrylic acid ester compounds containing a nitrogen atom, and cyclic (meth)acrylamide compounds.

Examples of the monofunctional (meth)acrylic polymerizable compound (C)-II containing no cyclic structure include chain (meth)acrylic acid ester compounds.

In view of the curability of the dental polymerizable composition and the water resistance of the cured product, preferred as monofunctional (meth)acrylic polymerizable compound (C)-I containing a cyclic structure are (meth)acrylic acid ester compounds containing aromatic rings, and alicyclic (meth)acrylic acid ester compounds. In view of reducing odor generation, (meth)acrylic acid ester compounds containing one or more aromatic rings are more preferred. In view of greater curability of the dental polymerizable composition and even superior water resistance of the cured product, (meth) acrylic acid ester compounds containing a plurality of aromatic rings are even more preferred.

Examples of (meth)acrylic acid ester compounds containing one aromatic ring include phenyl (meth)acrylate, benzyl (meth)acrylate, phenoxyethyl (meth)acrylate, phenoxybutyl (meth)acrylate, phenoxydiethylene glycol (meth) acrylate, phenoxypolyethylene glycol (meth)acrylate, 4-methylphenyl (meth)acrylate, 4-n-butylphenyl (meth)acrylate, 4-t-butylphenyl (meth)acrylate, 4-nonylphenyl (meth)acrylate, o-2-propenylphenyl (meth)acrylate, benzhydrol (meth) acrylate, and cumylphenol (meth)acrylate.

Examples of (meth)acrylic acid ester compounds containing two aromatic rings include o-phenylphenol (meth) acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth) acrylate, butoxylated o-phenylphenol (meth)acrylate, butoxylated m-phenylphenol (meth)acrylate, butoxylated p-phenylphenol (meth)acrylate, o-phenoxybenzyl (meth)acrylate, m-phenoxybenzyl (meth)acrylate, p-phenoxybenzyl (meth) acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 4-biphenylyl (meth)acrylate, 1-naphthyl (meth)acrylate, 2-naphthyl (meth)acrylate, anthryl (meth)acrylate, fluorenyl (meth)acrylate, and fluorenylmethyl (meth)acrylate.

Examples of the alicyclic (meth)acrylic acid ester compounds include 2-(1-adamantyl)propyl (meth)acrylate, 2-methyladamantyl-2-yl (meth)acrylate, 2-ethyladamantyl-2-yl (meth)acrylate, 2-n-propyladamantyl-2-yl (meth)acrylate, 2-isopropyladamantyl-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1-methylpropyl (meth)acrylate, and 1-(adamantan-1-yl)-1-ethylpropyl (meth) acrylate.

Examples the cyclic (meth)acrylic acid ester compounds containing a nitrogen atom include pentamethylpiperidinyl (meth)acrylate, tetramethylpiperidinyl (meth)acrylate, and 4-(pyrimidin-2-yl)piperazin-1-yl (meth)acrylate.

Examples of the cyclic (meth)acrylamide compounds include N-(meth)acryloylmorpholine, N-(meth)acryloylpyrrolidine, N-(meth)acryloylpiperidine, N-(meth)acryloyl-2-methylpiperidine, and N-(meth)acryloyl-2,2,6,6-tetramethylpiperidine.

In view of superior toughness and water resistance of the cured product of a dental polymerizable composition of the present invention, preferred as monofunctional (meth)acrylic polymerizable compound (C) are o-phenoxybenzyl acrylate, m-phenoxybenzyl acrylate, p-phenoxybenzyl acrylate, 2-(o-phenoxyphenyl)ethyl acrylate, 2-(m-phenoxyphenyl)ethyl acrylate, 2-(p-phenoxyphenyl)ethyl acrylate, 4-biphenylyl (meth)acrylate, 1-naphthyl (meth)acrylate, and 2-naphthyl (meth)acrylate, more preferably o-phenoxybenzyl acrylate, m-phenoxybenzyl acrylate, p-phenoxybenzyl acrylate, 2-(o-phenoxyphenyl)ethyl acrylate, 2-(m-phenoxyphenyl)ethyl acrylate, and 2-(p-phenoxyphenyl)ethyl acrylate, even more preferably o-phenoxybenzyl acrylate, m-phenoxybenzyl acrylate, 2-(o-phenoxyphenyl)ethyl acrylate, and 2-(m-phenoxyphenyl)ethyl acrylate.

The content of the monofunctional (meth)acrylic polymerizable compound (C) in a dental polymerizable composition of the present invention is preferably 1.0 to 60 mass% in total 100 mass% of the polymerizable compounds. In view of achieving low viscosity in the dental polymerizable composition without impairing strength, toughness, and water resistance when combined with other components, and providing excellent toughness and water resistance to the cured product, the content of monofunctional (meth)acrylic polymerizable compound (C) is more preferably 5.0 to 50 mass%, even more preferably 10 to 40 mass%, particularly preferably 15 to 30 mass%.

The monofunctional (meth)acrylic polymerizable compound (C) may be used alone, or two or more thereof may be used in combination.

In a certain embodiment, in view of providing even superior toughness and water resistance to the cured product when combined with other components, the content of monofunctional (meth)acrylic polymerizable compound (C) is preferably 1.0 to 60 mass%, more preferably 5.0 to 50 mass%, even more preferably 10 to 40 mass%, particularly preferably 15 to 30 mass% in total 100 mass% of the dental polymerizable composition.

In another preferred embodiment, the mass ratio of the content of monofunctional (meth)acrylic polymerizable compound (C) and that of polyfunctional (meth)acrylic polymerizable compound (A) is preferably (A)/(C) > 1. With the content of polyfunctional (meth)acrylic polymerizable compound (A) exceeding that of monofunctional (meth)acrylic polymerizable compound (C), it is possible to achieve even superior strength, toughness, and water resistance in the shaped article when combined with other components.

In still another preferred embodiment, the mass ratio of the content of monofunctional (meth)acrylic polymerizable compound (C) and the total content of polyfunctional (meth)acrylic polymerizable compound (A)-I and polyfunctional (meth)acrylic polymerizable compound (A)-II is preferably ((A)-I + (A)-II)/(C) > 1. With the total content of polyfunctional (meth)acrylic polymerizable compound (A)-I and polyfunctional (meth)acrylic polymerizable compound (A)-II exceeding the content of monofunctional (meth)acrylic polymerizable compound (C), it is possible to achieve even superior strength, toughness, and water resistance in the cured product.

The mass ratio ((A)-I + (A)-II)/(C) is more preferably 1.2 or more, even more preferably 2 or more, particularly preferably 3 or more.

The mass ratio ((A)-I + (A)-II)/(C) is preferably 20 or less, more preferably 15 or less, even more preferably 10 or less, particularly preferably 6 or less.

In certain embodiments, a dental polymerizable composition of the present invention may be essentially free of (meth)acrylamide group-containing compounds as polymerizable compounds.

Here, by "essentially free of (meth)acrylamide group-containing compounds", it means that the content of (meth) acrylamide group-containing compounds is less than 1 mass%, preferably less than 0.1 mass%, more preferably less than 0.01 mass%, even more preferably 0 mass% in the dental polymerizable composition.

[Photopolymerization Initiator (B) Containing no Phosphine Oxide Framework]

In a dental polymerizable composition of the present invention, the photopolymerization initiator (B) containing no phosphine oxide framework (hereinafter, also referred to simply as "photopolymerization initiator (B)") is used to impart desired curability to the dental polymerizable composition, and, in view of leachables from the cured product, it is essential that the photopolymerization initiator (B) does not contain a phosphine oxide framework. The photopolymerization initiator (B) may be selected from polymerization initiators used in industry. In view of leachables from the cured product, it is preferable to comprise a Norrish Type II photopolymerization initiator.

As used herein, "Norrish Type II photopolymerization initiator" refers to a photopolymerization initiator that is capable of generating radicals through a hydrogen abstraction radical mechanism.

Examples of the photopolymerization initiator (B) include $\alpha$-aminoketone compounds, oxime ester compounds, thioxanthones or quaternary ammonium salts of thioxanthones, benzophenones, oxyphenyl acetate ester compounds, $\alpha$-diketones, ketals, coumarins, anthraquinones, and benzoin alkyl ether compounds.

The photopolymerization initiator (B) may be used alone, or two or more thereof may be used in combination.

In view of enabling a further reduction of leachables from the cured product, the photopolymerization initiator (B) preferably comprises at least one selected from the group consisting of an $\alpha$-aminoketone compound, an oxime ester compound, a thioxanthone, and a quaternary ammonium salt of a thioxanthone. More preferably, the photopolymerization initiator (B) comprises at least one selected from the group consisting of an $\alpha$-aminoketone compound, an oxime ester compound, and a thioxanthone. Even more preferably, the photopolymerization initiator (B) comprises an $\alpha$-aminoketone compound and/or an oxime ester compound.

With the photopolymerization initiator (B), a dental polymerizable composition can be obtained that has excellent photocurability both in the ultraviolet and visible regions, and that shows sufficient photocurability even when the light source is a laser, a halogen lamp, a light emitting diode (LED), or a xenon lamp, reducing leaching of degradation products of the photopolymerization initiator from the cured product.

In a certain embodiment, in view of superior curability, the photopolymerization initiator (B) is preferably a compound with one or more aromatic rings, more preferably a compound with two or more aromatic rings.

Another preferred embodiment is, for example, a dental polymerizable composition in which the photopolymerization initiator (B) is a compound that does not comprise a terminal polymerizable group(s) in its structure.

Examples of the polymerizable groups include vinyl groups, allyl groups, (meth)acryloyl groups, and vinyl cycloalkyl groups (for example, vinyl cyclopropyl groups).

Examples of $\alpha$-aminoketone compounds that can be used as the photopolymerization initiator include 2-benzyl-2-(dimethylamino)-4'-morpholinobutyrophenone, and 2-methyl-1-[4-(methylthio)phenyl]-2-morpholinopropan-1-one.

Examples of oxime ester compounds that can be used as the photopolymerization initiator include 1,2-octanedione,1-[4-(phenylthio)phenyl]-,2-(o-benzoyloxime) (another name: 1-[4-(phenylthio)phenyl]octane-1,2-dione=2-(O-benzoyloxime)), and ethanone,1-[9-ethyl-6-(2-methylbenzoyl)-9H-carbazol-3-yl]-,1-(o-acetyloxime) (another name: Irgacure OXE-02).

Examples of thioxanthones or quaternary ammonium salts of thioxanthones that can be used as the photopolymerization initiator include thioxanthone, 2-chlorothioxanthone, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminium chloride, 2-hydroxy-3-(9-oxo-9H-thioxanthen-2-yloxy)-N,N,N-trimethyl-propaneaminium chloride, 2-hydroxy-3-(3,4-dimethyl-9-oxo-9H-thioxanthen-2-yloxy)-N,N,N-trimethyl-1-propaneaminium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propaneaminium chloride.

In view of enabling a further reduction of leachables from the cured product, 2-chlorothioxanthone is particularly preferred as a thioxanthone among these thioxanthones or quaternary ammonium salts of thioxanthones. Particularly preferred as a quaternary ammonium salt of a thioxanthone is 2-hydroxy-3-(3,4-dimethyl-9H-thioxanthen-2-yloxy)-N,N,N-trimethyl-1-propaneaminium chloride.

Examples of α-diketones that can be used as the photopolymerization initiator include diacetyl, dibenzyl, camphorquinone, 2,3-pentadione, 2,3-octadione, 9,10-phenanthrenequinone, 4,4'-oxybenzyl, and acenaphthenequinone.

Examples of ketals that can be used as the photopolymerization initiator include benzyl dimethyl ketal, and benzyl diethyl ketal.

Examples of coumarin compounds that can be used as the photopolymerization initiator include compounds mentioned in JP H09-3109 A and JP H10-245525 A, such as 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzol[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Examples of anthraquinones that can be used as the photopolymerization initiator include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Examples of benzoin alkyl ethers that can be used as the photopolymerization initiator include benzoin methyl ether, benzoin ethyl ether, benzoin isopropyl ether, and benzoin isobutyl ether.

In view of enabling a further reduction of leachables from the cured product, preferred are 2-benzyl-2-(dimethylamino)-4'-morpholinobutyrophenone, 2-methyl-1-[4-(methylthio)phenyl]-2-morpholinopropan-1-one, 1,2-octanedione,1-[4-(phenylthio)phenyl]-,2-(o-benzoyloxime), ethanone,1-[9-ethyl-6-(2-methylbenzoyl)-9H-carbazol-3-yl]-,1-(o-acetyloxime), 2-chlorothioxanthone, and 2-hydroxy-3-(3,4-dimethyl-9H-thioxanthen-2-yloxy)-N,N,N-trimethyl-1-propaneaminium chloride.

The content of the photopolymerization initiator (B) in a dental polymerizable composition of the present invention is not particularly limited. However, in view of curability and other properties of the dental polymerizable composition obtained, the content of photopolymerization initiator (B) is preferably 0.01 to 20 parts by mass relative to total 100 parts by mass of the polymerizable compounds. When the content of photopolymerization initiator (B) is less than 0.01 parts by mass, polymerization may not sufficiently proceed to form a shaped product. The content of photopolymerization initiator (B) is more preferably 0.05 parts or more by mass, even more preferably 0.1 parts or more by mass, particularly preferably 0.5 parts or more by mass relative to total 100 parts by mass of the polymerizable compounds. If the content of photopolymerization initiator (B) exceeds 20 parts by mass, the polymerization initiator itself blocks light, resulting in a dental polymerizable composition with inadequate curability. The content of photopolymerization initiator (B) is more preferably 15 parts or less by mass, even more preferably 10 parts or less by mass, particularly preferably 5.0 parts or less by mass relative to total 100 parts by mass of the polymerizable compounds.

A dental polymerizable composition of the present invention may comprise a photopolymerization initiator containing a phosphine oxide framework, for purposes such as adjusting its photocurability.

In view of leachability from the cured product, the content of the photopolymerization initiator containing a phosphine oxide framework is preferably 0.5 mass% or less, more preferably 0.3 mass% or less, even more preferably 0.1 mass% or less, particularly preferably 0 mass% (absent) in total 100 mass% of the dental polymerizable composition.

A dental polymerizable composition of the present invention may comprise a Norrish Type I photopolymerization initiator, for purposes such as adjusting its photocurability.

In view of leachability from the cured product, the content of the Norrish Type I (cleavage within the molecule) photopolymerization initiator is preferably 0.5 mass% or less, more preferably 0.3 mass% or less, even more preferably 0.1 mass% or less, particularly preferably 0 mass% (absent) in the dental polymerizable composition.

A dental polymerizable composition of the present invention is not particularly limited, and may comprise other components for example, as long as it comprises the polyfunctional (meth)acrylic polymerizable compound (A), and the photopolymerization initiator (B). The method of production of a dental polymerizable composition of the present invention is not particularly limited, and a dental polymerizable composition of the present invention can be produced following known methods of producing dental polymerizable compositions.

A dental polymerizable composition of the present invention may comprise a polymerization accelerator to improve photocurability, provided that it does not hinder the intent and purpose of the present invention.

Examples of the polymerization accelerator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of imparting superior curability to the dental polymerizable composition and enabling a further reduction of leachables from the cured product, preferred is at least one selected from the group consisting of ethyl 4-(N,N-dimethylamino)benzoate, n-butoxyethyl 4-(N,N-dimethylamino)benzoate, and 4-(N,N-dimethylamino)benzophenone.

A dental polymerizable composition of the present invention may further comprise a filler mixed therein to adjust paste properties or to improve the mechanical strength of the shaped article of the dental polymerizable composition. Examples of the filler include organic fillers, inorganic fillers, and organic-inorganic composite fillers. The filler may be used alone, or two or more thereof may be used in combination.

Examples of organic filler materials include polymethyl methacrylate, polyethyl methacrylate, a methyl methacrylate-ethyl methacrylate copolymer, crosslinked polymethyl methacrylate, crosslinked polyethyl methacrylate, polyesters, polyamides, polycarbonates, polyphenylene ethers, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used in combination. The shape of organic filler is not particularly limited, and the particle diameter of filler may be appropriately selected for use.

Examples of inorganic filler materials include quartz, silica, alumina, silica-titania, silica-titania-barium oxide, silica-zirconia, silica-alumina, lanthanum glass, borosilicate glass, soda glass, barium glass, strontium glass, glass-ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. These may be used alone, or two or more thereof may be used in combination. The shape of inorganic filler is not particularly limited, and may be appropriately selected from different types of fillers, such as irregularly shaped fillers, and spherical fillers.

The organic-inorganic composite fillers may be those produced using the organic fillers and inorganic fillers following known methods.

A dental polymerizable composition of the present invention may comprise a polymer to alter properties such as flexibility and flowability, provided that it does not hinder the intent and purpose of the present invention. Examples include natural rubber, synthetic polyisoprene rubber, liquid polyisoprene rubber and hydrogenated products thereof, polybutadiene rubber, liquid polybutadiene rubber and hydrogenated products thereof, styrene-butadiene rubber, chloroprene rubber, ethylene-propylene rubber, acryl rubber, isoprene-isobutylene rubber, acrylonitrile-butadiene rubber, and styrene elastomers. Specific examples of other polymers that may be added include a polystyrene-polyisoprene-polystyrene block copolymer, a polystyrene-polybutadiene-polystyrene block copolymer, a poly($\alpha$-methylstyrene)-polybutadiene-poly($\alpha$-methylstyrene) block copolymer, a poly(p-methylstyrene)-polybutadiene-poly(p-methylstyrene) block copolymer, and hydrogenated products of these.

A dental polymerizable composition of the present invention may optionally comprise a softener. Examples of the softener include petroleum-based softeners such as paraffinic, naphthenic, and aromatic process oils, and vegetable oil-base softeners such as paraffin, peanut oil, and rosin. These softeners may be used alone, or two or more thereof may be used in combination. The softener content is not particularly limited, provided that it does not hinder the intent and purpose of the present invention. Typically, the softener content is 200 parts or less by mass, preferably 100 parts or less by mass relative to total 100 parts by mass of the polymerizable compounds.

The content of the polymers and softeners is not particularly limited, as long as it does not hinder the intent and purpose of the present invention. The content of the polymers and softeners may be less than 5 mass%, less than 1 mass%, less than 0.1 mass%, or 0 mass% in total 100 mass% of the dental polymerizable composition.

A dental polymerizable composition of the present invention may comprise a known stabilizer, in order to inhibit deterioration or adjust photocurability. Examples of such stabilizers include polymerization inhibitors, ultraviolet absorbers, and antioxidants. The stabilizers may be used alone, or two or more thereof may be used in combination.

Examples of the polymerization inhibitors include hydroquinone, hydroquinone monomethyl ether, dibutylhydroquinone, dibutylhydroquinone monomethyl ether, 4-t-butyl catechol, 2-t-butyl-4,6-dimethylphenol, 2,6-di-t-butylphenol, and 3,5-di-t-butyl-4-hydroxytoluene. In view of color tone and curability, the content of the polymerization inhibitors is preferably 0.001 to 5.0 parts by mass, more preferably 0.01 to 1.0 parts by mass relative to total 100 parts by mass of the polymerizable compounds.

A dental polymerizable composition of the present invention may comprise a known additive, in order to adjust color tones or paste properties. Examples of such additives include colorants (pigments, dyes), organic solvents, and thickeners. The additive may be used alone, or two or more thereof may be used in combination.

The content of the additive (for example, an organic solvent) is not particularly limited, as long as it does not hinder the intent and purpose of the present invention. The additive content may be less than 5 mass%, less than 1 mass%, less than 0.1 mass%, or 0 mass% in total 100 mass% of the dental polymerizable composition.

A dental polymerizable composition of the present invention exhibits excellent strength, toughness, and water resistance in the cured product while reducing leachables, in addition to possessing excellent curability. This makes a dental polymerizable composition of the present invention usable in applications where such advantages can be exploited, including intraoral applications.

Examples of intraoral applications include dental materials such as dental adhesives, dental cements, dental restorative filling materials, denture base materials, and dental occlusal splints; and materials for treating sleep disorder (particularly, appliances for the treatment of sleep apnea). A dental polymerizable composition of the present invention is suited for stereolithographic materials, with optimum suitability for stereolithographically fabricated dental occlusal splints, denture base materials, and appliances for the treatment of sleep apnea.

An example of a preferred embodiment as a dental adhesive is as follows.

A preferred embodiment as a dental adhesive is, for example, a dental polymerizable composition that comprises a polyfunctional (meth)acrylic polymerizable compound (A), a photopolymerization initiator (B), a monofunctional (meth) acrylic polymerizable compound (C), and, optionally, a water-soluble organic solvent (such as water, ethanol, or acetone), a polymerization accelerator, and an inorganic filler.

Examples of the polyfunctional (meth)acrylic polymerizable compound (A) include 2,2-bis[4-(2-hydroxy-3-methacryloyloxypropoxy)phenyl]propane (commonly known as Bis-GMA), and glycerol di(meth)acrylate.

Examples of the monofunctional (meth)acrylic polymerizable compound (C) include a monofunctional (meth) acrylic polymerizable monomer containing an acidic group, and a monofunctional (meth)acrylic polymerizable monomer containing a hydroxyl group.

The dental adhesive is preferably one comprising 10 to 90 mass% of polyfunctional (meth)acrylic polymerizable compound (A), 0.001 to 10 mass% of photopolymerization initiator (B), 1.0 to 50 mass% of monofunctional (meth)acrylic polymerizable compound (C), 0 to 50 mass% of water, and 0 to 50 mass% of a water-soluble organic solvent within the total amount of the dental adhesive. More preferably, the dental adhesive is one comprising 20 to 80 mass% of polyfunctional (meth)acrylic polymerizable compound (A), 0.01 to 5.0 mass% of photopolymerization initiator (B), 5.0 to 40 mass% of monofunctional (meth)acrylic polymerizable compound (C), 5 to 40 mass% of water, and 5 to 40 mass% of a water-soluble organic solvent within the total amount of the dental adhesive. Even more preferably, the dental adhesive is one comprising 30 to 70 mass% of polyfunctional (meth)acrylic polymerizable compound (A), 0.1 to 1.0 mass% of photopolymerization initiator (B), 10 to 30 mass% of monofunctional (meth)acrylic polymerizable compound (C), 10 to 30 mass% of water, and 10 to 30 mass% of a water-soluble organic solvent within the total amount of the dental adhesive.

A cured product using a dental polymerizable composition of the present invention may have a shape that depends on intended use. In a dental polymerizable composition of the present invention, the type and content of polyfunctional (meth)acrylic polymerizable compound (A) and photopolymerization initiator (B), as well as optional components (such as monofunctional (meth)acrylic polymerizable compound (C), polymerization accelerators, fillers, polymers, softeners, stabilizers, and additives) may be optionally adjusted for different uses, for example, dental occlusal splints, denture base materials, and appliances for the treatment of sleep apnea.

A dental polymerizable composition of the present invention can be used in a wide variety of applications by taking advantage of its properties, specifically, the superior shape precision due to the low rate of volume shrinkage upon curing with light, and the ability to produce shaped products, three-dimensional shaped articles, or other shaped articles that exhibit superior strength, toughness, and water resistance in cured form.

For example, a dental polymerizable composition of the present invention can be used for stereolithographical production of three-dimensional shaped articles, and production of various shaped products, for example, film-shaped articles or molded articles produced by a technique such as film casting or casting, and molds for coating and vacuum molding applications.

A dental polymerizable composition of the present invention is particularly suited for stereolithography such as above. In stereolithography applications, a dental polymerizable composition of the present invention enables smooth production of a three-dimensional shaped article having superior toughness and mechanical characteristics while ensuring superior dimensional accuracy with a maintained low rate of volume shrinkage at the time of curing with light.

A dental polymerizable composition of the present invention exhibits excellent strength, toughness, and water resistance, and possesses adequate flexibility with mechanical characteristics that are not excessively high, in addition to achieving reduced leachables. This makes a dental polymerizable composition of the present invention suitable in intraoral applications, particularly as orthodontic mouthpieces or aligners used to adjust tooth alignment and/or jaw position, or dental soft splints such as mouthguards used in sports.

Another embodiment of the present invention is a method for producing a three-dimensional shaped article by a stereolithography method using any of the dental polymerizable compositions described above. The stereolithography method is not particularly limited, and may employ vat photopolymerization techniques such as laser SLA (Stereolithography Apparatus), and DLP (digital light processing) SLA. An example of applicable SLA techniques is LFS (Low Force Stereolithography).

In stereolithography using a dental polymerizable composition of the present invention, any known stereolithography method and device (for example, a stereolithography device such as the DIGITALWAX® 028J-Plus manufactured by DWS) may be used. In the present invention, the light energy used to cure the resin is preferably an active energy beam. As used herein, "active energy beam" means an energy ray capable of curing a dental polymerizable composition, and includes, for example, ultraviolet light, an electron beam, X-rays, radiant rays, and high-frequency waves. For example, the active energy beam may be ultraviolet light of 300 to 400 nm wavelengths. The light source of active energy beam may be, for example, a laser such as an Ar laser or a He-Cd laser; or a lighting such as a halogen lamp, a xenon lamp, a metal halide lamp, an LED, a mercury lamp, or a fluorescent lamp. Lasers are particularly preferred. When the light source is a laser, the fabrication time can be reduced by increasing the energy level, and a three-dimensional shaped article of high shape precision can be obtained by taking advantage of the desirable convergence of a laser beam.

Stereolithography using a dental polymerizable composition of the present invention may use any known method and any known stereolithography system, and the method and device are not particularly limited, as noted above. However, a typical example of a stereolithography method preferred for use in the present invention is a method that produces the desired final three-dimensional shaped article through a repeated procedure that includes a step of forming a cured layer by selectively applying an active energy beam to the dental polymerizable composition so as to obtain a cured layer having a desired pattern, and a step of continuously forming another cured layer on the previously formed cured layer by similarly applying an active energy beam to a newly supplied, uncured liquid of the dental polymerizable composition. The resulting three-dimensional shaped article may be used as is, or, depending on the application, it may be used after improving mechanical characteristics, shape stability, or other properties by, for example, post-curing the product under applied light or heat.

The three-dimensional shaped article obtained by stereolithography is not limited to a particular structure, shape, or size, and these may be decided according to use. Typical examples of areas to which the stereolithography method of the present invention is applicable include production of various models and molds, including, for example, models for assessing external designs in a designing process; models for checking functions of components or parts; resin molds for making casting molds; base models for making molds; and direct molds for prototype molds. More specifically, the stereolithography method of the present invention is applicable to, for example, production of models or work models for precision components and parts, electrical components, electronic components, furniture, architectural structures, automobile parts, various containers and vessels, castings, molds, and masters. By taking advantage of the excellent strength and toughness of the shaped article of the dental polymerizable composition, the stereolithography method of the present invention can be very effectively used for, for example, complex-shape cushioning materials in structures (for example, architectural structures), and molds for vacuum molding.

EXAMPLES

The following describes the present invention in greater detail by way of Examples. It should be noted, however, that the present invention is in no way limited by the following Examples, and various changes may be made by a person with ordinary skill in the art within the technical idea of the present invention.

The components used for the dental polymerizable compositions according to Examples and Comparative Examples are described below, along with the abbreviations.

[Polyfunctional Urethanized (Meth)Acrylic Polymerizable Compound (A)-I-1]

UDMA: 2,2,4-trimethylhexamethylenebis(2-carbamoyloxyethyl)dimethacrylate (manufactured by Kyoeisha Chemical Co., Ltd.; molecular weight: 471)
U4TH: N,N'-(2,2,4-trimethylhexamethylene)bis[2-(aminocarboxy)propane-1,3-diol]tetramethacrylate (manufactured by Kyoeisha Chemical Co., Ltd.; molecular weight: 673)

[Polyfunctional (Meth)Acrylic Polymerizable Compound (A)-I-2 Containing no Urethane Bond]

D2.6E: 2,2-bis(4-methacryloyloxypolyethoxyphenyl)propane (with an average of 2.6 moles of ethoxy group added; BPE-100 manufactured by Shin-Nakamura Chemical Co., Ltd.; molecular weight: approximately 478)

[Polyfunctional Urethanized (Meth)Acrylic Polymerizable Compound (A)-II-1]

<Synthesis Example 1> [Production of Polyfunctional Urethanized (Meth)Acrylic Polymerizable Compound (A)-II-1-a]

(1) A 5 L four-neck flask equipped with a stirrer, a thermostat, a thermometer, and a condenser was charged with 250 g of isophorone diisocyanate and 0.15 g of di-n-butyltin dilaurate, and the mixture was heated to 70°C while being stirred.

(2) Separately, 2,500 g of polyester polyol (Kuraray Polyol® P-2050 manufactured by Kuraray Co., Ltd.; a polyol composed of sebacic acid and 3-methyl-1,5-pentanediol; weight-average molecular weight Mw: 2,000) was added into a dropping funnel equipped with a side tube, and the solution in the dropping funnel was dropped into the flask of (1). Here, the solution was dropped at a constant rate over a time period of 4 hours with the temperature inside the flask held at 65 to 75°C while stirring the solution in the flask of (1). After dropping, the mixture was stirred at the same temperature for 2 hours to allow reaction.

(3) Thereafter, a homogenous solution prepared by adding 150 g of 2-hydroxyethyl acrylate and 0.4 g of hydroquinone monomethyl ether into a different dropping funnel was dropped into the flask of (2) at a constant rate over a time period of 2 hours with the temperature inside the flask held at 55 to 65°C, and a reaction was allowed for 4 hours at the maintained solution temperature of 70 to 80°C in the flask to obtain a urethanized (meth)acrylic polymerizable compound (A)-II-1-a. The polyfunctional urethanized (meth)acrylic polymerizable compound (A)-II-1-a had a weight-average molecular weight Mw of 2,600 as measured by GPC analysis.

The weight-average molecular weight Mw of the compound synthesized above means a weight-average molecular weight in terms of polystyrene equivalents, as determined by gel permeation chromatography (GPC).

Polyfunctional urethanized (meth)acrylic polymerizable compound (A)-II-1-b: polyether-based urethane acrylate; molecular weight: 1,600; NK Oligo UA-160TM manufactured by Shin-Nakamura Chemical Co., Ltd.

[Polyfunctional (Meth)Acrylic Polymerizable Compound (A)-II-2 Containing no Urethane Bond]

A-BPE-30: 2,2-bis(4-methacryloyloxypolyethoxyphenyl)propane (with an average of 30 moles of ethoxy group added; A-BPE-30 manufactured by Shin-Nakamura Chemical Co., Ltd.; molecular weight: approximately 1,656)

[Monofunctional (Meth)Acrylic Polymerizable Compound (C)]

POBA: m-phenoxybenzyl acrylate (manufactured by Kyoeisha Chemical Co., Ltd.)
A-LEN-10: ethoxylated o-phenylphenol acrylate (manufactured by Shin-Nakamura Chemical Co., Ltd.)

[Photopolymerization Initiator (B) Containing no Phosphine Oxide Framework]

AK1: 2-benzyl-2-(dimethylamino)-4'-morpholinobutyrophenone (Omnirad 369, manufactured by IGM Resin B.V. (NL))
OX1: 1,2-octanedione,1-[4-(phenylthio)phenyl]-,2-(o-benzoyloxime) (Irgacure OXE-01, manufactured by BASF Japan)
CT1: 2-chlorothioxanthone (manufactured by Tokyo Chemical Industry Co., Ltd.)

[Photopolymerization Initiator Containing Phosphine Oxide Framework]

TPO: diphenyl(2,4,6-trimethylbenzoyl)phosphine oxide
BAPO: phenylbis(2,4,6-trimethylbenzoyl)phosphine oxide

[Polymerization Inhibitor]

BHT: 3,5-di-t-butyl-4-hydroxytoluene

[Examples 1 to 13 and Comparative Examples 1 and 2]

The components were mixed at an ordinary temperature (20°C $\pm$ 15°C; JIS (Japanese Industrial Standards) Z 8703:1983) in the amounts (parts by mass) shown in Tables 1 and 2 to prepare dental polymerizable compositions according to Examples 1 to 13 and Comparative Examples 1 and 2.

The dental polymerizable composition was fabricated into a specimen with the dimensions (64.0 mm in length, 10.0 mm in width, and 3.3 mm in thickness) described in JDMAS 253:2020 (3D printing photopolymerizable resin for dental hard splints) 6.4, using a stereolithography device (DIGITALWAX® 020D manufactured by DWS). Various tests were conducted using this specimen as a cured product of the dental polymerizable composition.

<Strength (Flexural Modulus, Flexural Strength), Toughness (Displacement of Flexural Fracture Point)>

The cured products of the dental polymerizable compositions according to Examples and Comparative Examples were stored in air for 1 day, and measured for strength (flexural modulus, flexural strength) and toughness (displacement of flexural fracture point). In the measurement, a flexural strength test was conducted in compliance with JDMAS 253:2020 (3D printing photopolymerizable resin for dental hard splints) 6.4 at a span length of 50 mm and a crosshead speed of 5 mm/min, using a universal testing machine (Autograph AG-I 100kN manufactured by Shimadzu Corporation) (n = 5). The mean values of the measurements are presented in Tables 1 and 2.

In view of applicability particularly suited as dental occlusal splints, the preferred flexural modulus range is 300 MPa or more and less than 2,000 MPa, more preferably 400 MPa or more and 1,500 MPa or less, even more preferably 500 MPa or more and 1,000 MPa or less.

The preferred flexural strength (three-point flexural strength) is 10 MPa or more, more preferably 15 MPa or more, even more preferably 20 MPa or more.

As for the displacement of fracture point, it is preferable to have no fracture.

The displacement of fracture point was evaluated based on the following criteria, with ratings "A" (favorable toughness) and "B" (moderate toughness) considered acceptable.

<Evaluation Criteria>

A: The cured product fractured at a displacement of 15 mm or more.
B: The cured product fractured at a displacement of more than 10 mm and less than 15 mm.
C: The cured product fractured at a displacement of 10 mm or less.

<Water Resistance>

The cured products of the dental polymerizable compositions according to Examples and Comparative Examples were immersed in 37°C water for 168 hours, and measured for flexural strength in the same manner as in the flexural strength test above (n = 5). The mean values of the measurements are presented in Tables 1 and 2. Assuming that the measured flexural strength (unit: MPa) in the flexural strength test above is the "initial flexural strength", a rate of change (percentage decrease) in flexural strength of 20% or less indicates excellent water resistance after 1 day of storage in air and subsequent immersion in 37°C water for 168 hours, as indicated below. In Tables 1 and 2, the flexural strength after immersion in 37°C water for 168 hours is denoted as "Flexural strength after immersion."

Rate of change (percentage decrease) in flexural strength (%) = [{initial flexural strength (MPa) - flexural strength after immersion in 37°C water for 168 hours (MPa)} / initial flexural strength (MPa)] $\times$ 100

<Leachability>

The dental polymerizable composition obtained was used to fabricate circular discs each measuring 15 mm in diameter and 1.0 mm in thickness, using a stereolithography device (DIGITALWAX® 020D, manufactured by DWS). A sample disc was then immersed in 2.0 ml of ethanol in a 10 ml glass vial at 50°C for 72 hours. After immersion, the circular disc was removed, and the ethanol was distilled away before measuring the mass of the resulting extract (n = 1).

The extract was then dissolved in THF, and analyzed to identify its main components by GC-MS (instrument: GCMS-QP2010 Ultra manufactured by Shimadzu Corporation; column: Ultra ALLOY-1 manufactured by Frontier Laboratories Ltd., 0.25 mm in inner diameter, 15 m in length, and 0.15 $\mu$m in thickness).

[Table 1]

| Components (parts by mass) | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|---|
| Raw materials | (A)-I | UDMA | 45 | 45 | 45 | 45 | 45 | 35 | 55 | 45 | 40 | | 45 | 45 | 45 |
| | | U4TH | | | | | | | | | 5 | | | | |
| | | D2.6E | | | | | | | | | | 45 | | | |
| | (A)-II | Polyfunctional urethanized (meth)acrylic monomer (A)-II-1-a | 35 | 35 | 35 | 35 | 35 | 40 | 30 | 35 | 35 | 35 | | | 35 |
| | | Polyfunctional urethanized (meth)acrylic monomer (A)-II-1-b | | | | | | | | | | | 35 | | |
| | | A-BPE-30 | | | | | | | | | | | | 35 | |
| | (C) | POBA | 20 | 20 | 20 | 20 | 20 | 25 | | | 20 | 20 | 20 | 20 | 20 |
| | | | | | | | | | | 20 | | | | | |
| | (B) | AK1 | 2.0 | 1.0 | 3.0 | | | 2.0 | 2.0 | 2.0 | 2.0 | 2.0 | 2.0 | 2.0 | 2.0 |
| | | OX1 | | | | 2.0 | | | | | | | | | |
| | | CT1 | | | | | 2.0 | | | | | | | | |
| | | TPO | | | | | | | | | | | | | 0.09 |
| | | BHT | 0.5 | 0.1 | 1.0 | 0.5 | 0.5 | 0.5 | 0.5 | 0.5 | 0.5 | 0.5 | 0.5 | 0.5 | 0.5 |
| Properties | Strength | Flexural strength (MPa) | 28 | 28 | 27 | 27 | 27 | 23 | 32 | 29 | 33 | 30 | 31 | 27 | 28 |
| | | Flexural modulus (MPa) | 750 | 740 | 770 | 740 | 730 | 670 | 840 | 780 | 860 | 780 | 820 | 760 | 760 |
| | Toughness | Displacement of fracture point | | A | A | A | A | A | A | A | A | B | B | A | B | A |
| | Water resistance | Flexural strength after immersion (MPa) | 27 | 27 | 26 | 26 | 26 | 21 | 31 | 27 | 32 | 26 | 29 | 23 | 28 |
| | | Percentage decrease (%) | 3.6 | 3.6 | 3.7 | 3.7 | 3.7 | 8.7 | 3.1 | 6.9 | 3.0 | 13.0 | 6.5 | 15.0 | 0.0 |
| | Amount of leachables | mg | 0.2 | 0.3 | 0.1 | 0.2 | 0.2 | 0.3 | 0.1 | 0.3 | 0.1 | 0.4 | 0.2 | 0.5 | 0.6 |

[Table 2]

| Components (parts by mass) | | | | Com. Ex. 1 | Com. Ex. 2 |
|---|---|---|---|---|---|
| Raw materials | (A)-I | UDMA | | 45 | 35 |
| | | U4TH | | | |
| | | D2.6E | | | |
| | (A)-II | Polyfunctional urethanized (meth)acrylic monomer (A)-II-1-a | | 35 | 35 |
| | | Polyfunctional urethanized (meth)acrylic monomer (A)-II-1-b | | | |
| | | A-BPE-30 | | | |
| | (C) | POBA | | 20 | 25 |
| | | A-LEN-10 | | | |
| | (B) | AK1 | | | |
| | | OX1 | | | |
| | | CT1 | | | |
| | | TPO | | 2.0 | |
| | | BAPO | | | 2.0 |
| | | BHT | | 0.5 | 0.5 |
| Properties | Strength | Flexural strength | (MPa) | 28 | 27 |
| | | Flexural modulus | (MPa) | 760 | 780 |
| | Toughness | Displacement of fracture point | | A | A |
| | Water resistance | Flexural strength after immersion | (MPa) | 27 | 26 |
| | | Percentage decrease | (%) | 3.6 | 3.6 |
| | Amount of leachables | | mg | 2.8 | 3.1 |

As indicated in Tables 1 and 2, the shaped articles of the dental polymerizable compositions of Examples 1 to 13 exhibited excellent strength, toughness, and water resistance, and released fewer leachables than the shaped articles of the dental polymerizable compositions of Comparative Examples 1 and 2.

In addition, GC-MS analysis of leachables from the shaped articles of the dental polymerizable compositions of Examples 1 to 13 detected only small quantities, and the components were not readily identifiable.

In contrast, analysis of leachables from the shaped articles of the dental polymerizable compositions of Comparative Examples 1 and 2 revealed the presence of trimethylbenzaldehyde, trimethylbenzoic acid, and diphenylphosphine derivatives. These results confirmed that the dental polymerizable compositions of Examples 1 to 13 release notably small quantities of leachables and have a higher level of safety compared with Comparative Examples 1 and 2.

INDUSTRIAL APPLICABILITY

A dental polymerizable composition of the present invention exhibits excellent strength, toughness, and water resistance in the cured product while reducing leachables from the cured product. This makes a dental polymerizable composition of the present invention optimal for intraoral applications, such as a variety of dental materials (particularly, dental occlusal splints and denture base materials fabricated by stereolithography) or various materials for treating sleep disorder (particularly, appliances for treating sleep apnea).

**Claims**

1. A dental polymerizable composition comprising a polyfunctional (meth)acrylic polymerizable compound (A), and a photopolymerization initiator (B) containing no phosphine oxide framework.

2. The dental polymerizable composition according to claim 1, wherein the photopolymerization initiator (B) containing no phosphine oxide framework comprises a Norrish Type II photopolymerization initiator.

3. The dental polymerizable composition according to claim 1 or 2, wherein the polyfunctional (meth)acrylic polymerizable compound (A) comprises a polyfunctional (meth)acrylic polymerizable compound (A)-I with a molecular weight of less than 1,000, and/or a polyfunctional (meth)acrylic polymerizable compound (A)-II with a molecular weight of 1,000 or more.

4. The dental polymerizable composition according to claim 3, wherein the polyfunctional (meth)acrylic polymerizable compound (A) comprises a polyfunctional urethanized (meth)acrylic polymerizable compound (A)-I-1 that contains a urethane bond and has a molecular weight of less than 1,000, and/or a polyfunctional urethanized (meth)acrylic polymerizable compound (A)-II-1 that contains a urethane bond and has a molecular weight of 1,000 or more.

5. The dental polymerizable composition according to claim 4, wherein the polyfunctional (meth)acrylic polymerizable compound (A)-I-1 comprises a (meth)acrylate that contains no polymeric structure within a single molecule.

6. The dental polymerizable composition according to claim 4, wherein the polyfunctional urethanized (meth)acrylic polymerizable compound (A)-II-1 comprises a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

7. The dental polymerizable composition according to claim 4, wherein the polyfunctional urethanized (meth)acrylic polymerizable compound (A)-II-1 comprises a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

8. The dental polymerizable composition according to claim 1 or 2, wherein the photopolymerization initiator (B) containing no phosphine oxide framework comprises at least one selected from the group consisting of an α-aminoketone compound, an oxime ester compound, and a thioxanthone.

9. The dental polymerizable composition according to claim 1 or 2, which further comprises a monofunctional (meth)acrylic polymerizable compound (C).

10. The dental polymerizable composition according to claim 9, wherein the monofunctional (meth)acrylic polymerizable compound (C) comprises a monofunctional (meth)acrylic polymerizable compound (C)-I containing a cyclic structure.

11. The dental polymerizable composition according to claim 10, wherein the monofunctional (meth)acrylic polymerizable compound (C)-I comprises one or more aromatic rings.

12. The dental polymerizable composition according to claim 10, wherein the monofunctional (meth)acrylic polymerizable compound (C)-I comprises a plurality of aromatic rings.

13. A stereolithographic material comprising a dental polymerizable composition of claim 1 or 2.

14. A denture base material comprising a cured product of a stereolithographic material of claim 13.

15. A dental occlusal splint comprising a cured product of a stereolithographic material of claim 13.

16. A material for treating sleep disorder, comprising a cured product of a stereolithographic material of claim 13.

17. A method for stereolithographically producing a three-dimensional shaped article using a dental polymerizable composition of claim 1 or 2.

## INTERNATIONAL SEARCH REPORT

| International application No. |
|---|
| **PCT/JP2024/023620** |

| **A.** | **CLASSIFICATION OF SUBJECT MATTER** |
|---|---|

*A61K 6/60*(2020.01)i; *A61K 6/62*(2020.01)i; *A61K 6/887*(2020.01)i; *C08F 2/44*(2006.01)i; *C08F 290/06*(2006.01)i
FI:    A61K6/60; A61K6/62; A61K6/887; C08F2/44 Z; C08F290/06

According to International Patent Classification (IPC) or to both national classification and IPC

| **B.** | **FIELDS SEARCHED** |
|---|---|

Minimum documentation searched (classification system followed by classification symbols)

A61K6/60; A61K6/62; A61K6/887; C08F2/44; C08F290/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | WO 2020/153269 A1 (MITSUI CHEMICALS, INC.) 30 July 2020 (2020-07-30) | 1-5, 8, 9, 14 |
|   | Claims 1, 3, 5, 8, 15, paragraphs [0073]-[0075], [0104], examples 1-14, table 1 | |
| Y | | 1-17 |
| X | JP 60-149603 A (KURARAY CO., LTD.) 07 August 1985 (1985-08-07) | 1-5, 9 |
|   | Claim 1, page 3, upper left column, line 12 to lower right column, 7th line from the bottom, page 6, right column, lines 2-9, examples 1-10 | |
| Y | | 1-17 |
| X | US 2005/0020696 A1 (MONTGOMERY, R. E.) 27 January 2005 (2005-01-27) | 1-4, 6, 8, 9 |
|   | Claim 1, paragraphs [0048]-[0058], example II | |
| Y | | 1-17 |
| X | JP 2008-081446 A (GC DENTAL PRODUCTS CORPORATION) 10 April 2008 (2008-04-10) | 1-5, 8-10 |
|   | Claims 1, 4, paragraphs [0020]-[0022], examples 1-9 | |
| Y | | 1-17 |

| ✓ Further documents are listed in the continuation of Box C. | ✓ See patent family annex. |
|---|---|

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2024** | **10 September 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

| International application No. |
| --- |
| **PCT/JP2024/023620** |

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2007-223955 A (TOKUYAMA CORPORATION) 06 September 2007 (2007-09-06)<br>    Claims 1, 3, paragraph [0024], examples 1-11 | 1-5, 8-10 |
| Y | | 1-17 |
| X | JP 04-366113 A (KANEBO LTD.) 18 December 1992 (1992-12-18)<br>    Claim 1, paragraphs [0001], [0010], examples 1, 2 | 1-3, 5, 8, 9 |
| Y | | 1-17 |
| X | JP 02-138106 A (G-C TOSHI KOGYO CORPORATION) 28 May 1990 (1990-05-28)<br>    Claims 1, 7, page 2, upper left column 3rd line from the bottom to right column, line 5,<br>    page 4, lower left column line 11 to page 5, left column, page 5, lower left column, page<br>    6, upper right column, line 2, examples 1-18 | 1-3, 5, 8, 9, 14, 15 |
| Y | | 1-17 |
| Y | WO 2020/235628 A1 (KURARAY NORITAKE DENTAL INC.) 26 November 2020<br>(2020-11-26)<br>    Claims, paragraphs [0004], [0018]-[0041], [0083] | 1-17 |
| Y | WO 2021/162007 A1 (KURARAY NORITAKE DENTAL INC.) 19 August 2021<br>(2021-08-19)<br>    Claims, paragraphs [0003], [0016]-[0035] | 1-17 |

Form PCT/ISA/210 (second sheet) (July 2022)

**INTERNATIONAL SEARCH REPORT**
Information on patent family members

| International application No. |
| PCT/JP2024/023620 |

| Patent document cited in search report | | | Publication date (day/month/year) | Patent family member(s) | | | Publication date (day/month/year) |
|---|---|---|---|---|---|---|---|
| WO | 2020/153269 | A1 | 30 July 2020 | US | 2022/0087906 | A1 | |
| | | | | Claims 1, 3, 5, 8, 15, paragraphs [0141]-[0146], [0209], examples 1-14, table 1 | | | |
| | | | | EP | 3895681 | A1 | |
| JP | 60-149603 | A | 07 August 1985 | US | 4668712 | A | |
| | | | | Claim 1, column 2, line 22 to column 3, line 20, column 7, lines 31-45, examples 1-10 | | | |
| | | | | EP | 150952 | A2 | |
| US | 2005/0020696 | A1 | 27 January 2005 | WO | 2001/081437 | A1 | |
| JP | 2008-081446 | A | 10 April 2008 | (Family: none) | | | |
| JP | 2007-223955 | A | 06 September 2007 | (Family: none) | | | |
| JP | 04-366113 | A | 18 December 1992 | (Family: none) | | | |
| JP | 02-138106 | A | 28 May 1990 | US | 5063255 | A | |
| | | | | Claims 1, 7, column 1, lines 7-16, column 4, line 43 to column 5, line 17, column 5, line 60 to column 6, line 39, examples 1-18 | | | |
| | | | | GB | 2225333 | A | |
| | | | | DE | 3938359 | A | |
| WO | 2020/235628 | A1 | 26 November 2020 | US | 2022/0251275 | A1 | |
| | | | | Claims, paragraphs [0004], [0042]-[0067], [0111] | | | |
| | | | | EP | 3974455 | A1 | |
| WO | 2021/162007 | A1 | 19 August 2021 | US | 2023/0091071 | A1 | |
| | | | | Claims, paragraphs [0003], [0023]-[0047] | | | |
| | | | | EP | 4105250 | A1 | |
| | | | | CN | 115038730 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023074620 A
- JP H093109 A
- JP H10245525 A